# EUROPÄISCHE PATENTSCHRIFT

(11) **EP 2 149 336 B1**
(45) Veröffentlichungstag und Bekanntmachung des Hinweises auf die Patenterteilung: **22.08.2018**
(21) Anmeldenummer: 09163885.8
(22) Anmeldetag: 26.06.2009
(51) Int. Cl.: A61B 5/00, A61B 5/0285, A61B 5/0295, A61B 5/05, A61N 1/365

(54) **Vorrichtung und Verfahren zur Erfassung der Strömungsgeschwindigkeit eines Blutstromes und Herz-/Kreislauf-Unterstützungsvorrichtung**
Method and device for recording the flow rate of a blood flow and heart/circulation support device
Dispositif et procédé de saisie de la vitesse d'écoulement d'un flux de sang et dispositif de soutien cardio-vasculaire

(30) Priorität: 28.07.2008 DE 102008040788
(43) Veröffentlichungstag der Anmeldung: 03.02.2010
(73) Patentinhaber: BIOTRONIK SE & Co. KG, 12359 Berlin (DE)
(72) Erfinder: Kolberg, Gernot, 12043 Berlin (DE); Bartels, Klaus, 10115 Berlin (DE)
(74) Vertreter: Keck, Hans-Georg

(56) Entgegenhaltungen:
- WO-A-95/26677
- WO-A-2007/028035
- DE-A1- 19 507 929
- US-A- 5 602 342
- US-A1- 2008 058 630
- US-B2- 7 011 633

## Beschreibung

Die Erfindung betrifft eine Vorrichtung und ein Verfahren zur in-vivo-Erfassung der Strömungsgeschwindigkeit eines Blutstromes in einem Blutgefäß. Sie betrifft des Weiteren eine Herz-/Kreislauf-Unterstützungsvorrichtung, die eine solche Erfassungsvorrichtung umfasst.

Da ein mit hinreichender Geschwindigkeit zirkulierender Blutstrom entscheidend für die Vitalität eines Menschen oder auch höherer Tierarten ist, die als Haustiere gehalten werden, kommt der in-vivo-Erfassung der Strömungsgeschwindigkeit von Blut in der Human- und Veterinärmedizin erhebliche Bedeutung zu. Speziell kann sie von großem Wert für die Erfassung von lebensbedrohlichen Reizzuständen des Herzens und somit für die Steuerung von Geräten zur Unterstützung der Herz-/Kreislauffunktion, wie etwa Herzschrittmachern oder Defibrillatoren, sein.

Aus der Technik ist eine Vielzahl Fühler für die Erfassung von Strömungsgeschwindigkeiten bekannt, die auf unterschiedlichsten Messprinzipien basieren und zu einem großen Teil auch in der Praxis eingesetzt werden. Hierzu gehören etwa Sensoren auf Basis des Venturi- oder Bernoulliprinzips oder des Prinzips des Prandtlschen Staurohrs oder auch Doppler-sonografische Messanordnungen. Alle diese Anordnungen sind technisch relativ aufwendig und die meisten davon im lebenden Organismus in ihrer Funktionsfähigkeit durch das Aufwachsen von Zellen auf Sensorkomponenten und ähnliche Prozesse gefährdet. Doppler-sonografische Messvorrichtungen sind zudem energieaufwendig und schon von daher für einen Betrieb im implantierten Zustand nur wenig geeignet. Derartige Fühler sind z.B. aus den Dokumenten US 5 602 342, US 7 011 633, US 2008/058630, DE 195 07 929, WO 95/26677 und WO 2007/028035 bekannt. Auf indirektem Wege lassen sich Aussagen über die Bewegung des Blutes im lebenden Organismus auch mittels Drucksensoren oder weiterer Sensoren gewinnen, welche blutströmungsabhängige Größen erfassen. Hierzu zählen die sogenannten hämodynamischen Sensoren (HDS) oder Lösungen, welche intrakardiale Elektrogramme (IEGM) erfassen. Derartige Lösungen sind häufig relativ ungenau und störanfällig.

Aus S. Gawad: "Micromachined impedance spectroscopy flow cytometer for cell analysis and particle sizing", Lab on a Chip, 2001, 1, 76-82, ist ein Gerät zur Klassifizierung und Sortierung von Zellen oder Teilchen in einem Flüssigkeitsstrom aufgrund ihrer Größe bekannt, welches sich einer Mikroelektrodenanordnung bedient, mit der in ein Flüssigkeitsrohr eine elektrische Anregungsenergie eingebracht und ein daraufhin auftretendes Impedanzsignal erfasst wird, welches für die hindurchströmenden Zellen bzw. Teilchen charakteristisch ist.

Es sind auch Anordnungen zur Messung des Blutstromes im Herzen oder in Blutgefässen bekannt geworden, die zwei voneinander beabstandete Elektroden auf einer Elektrodenleitung bzw. einem Katheter, von denen eine als eine polarisierbare Kathode ausgeführt ist, zur Ausbildung und zugleich zur Erfassung eines galvanischen Zellepotentials nutzen, welches grundsätzlich von der Strömungsgeschwindigkeit des Blutes in die Umgebung der Elektroden abhängig ist. Eine entsprechende Anordnung, die zur Messung der Blutströmungsgeschwindigkeit im Bereich der Herzklappen in das Herz einsetzbar ist, beschreibt die US 5,799,350. Eine ähnliche Messanordnung, die sich als Messelektroden der Stimulationselektroden einer Schrittmacherleitung bedient, ist auch aus der US 5,602,342 bekannt. Eine ähnliche Messanordnung, die (wie auch die vorbeschriebene Anordnung) in einem Herzstimulationsgerät einsetzbar ist, beschreibt die US 7,011,633 B2. Diese Anordnung bedient sich als Messelektrode einer ringförmigen bzw. zylindrischen oder auch spiralig gewickelten Flächenelektrode, deren Innenoberfläche als aktive Messelektrodenfläche dient.

Die letztgenannten Anordnungen haben keinen Eingang in die Schrittmachertherapie gefunden, was vermutlich an der ungenügenden Messgenauigkeit liegt.

Der Erfindung liegt die Aufgabe zugrunde, eine verbesserte Vorrichtung zur in-vivo-Erfassung der Strömungsgeschwindigkeit eines Blutstromes in einem Blutgefäß anzugeben, welche insbesondere vergleichsweise unaufwendig sind und auch über längere Zeiträume hinreichend genau und zuverlässig funktionieren.

Diese Aufgabe wird durch eine Vorrichtung mit den Merkmalen des Anspruchs 1 gelöst. Zweckmäßige Ausgestaltungen des Erfindungsgedankens sind Gegenstand der abhängigen Ansprüche. Eine auf dieser Lösung aufbauende Herz-/Kreislauf-Unterstützungsvorrichtung ist Gegenstand des Anspruchs 14.

Nach Erkenntnissen der Erfinder ist mit den bekannten Messanordnungen, soweit diese auf einem elektrischen Messprinzip basieren, keine hinreichend genaue Messung möglich, weil diese nicht hinreichend zwischen dem signalgebenden Effekt der Konvektion und dem signalverfälschenden Effekt der ungerichteten Diffusion auf der Messstrecke zwischen der eingesetzten Elektrode differenzieren können. Die Erfinder haben erkannt, dass insbesondere die Nutzung der auf Schrittmacherleitungen ohnehin vorgesehenen, relativ großflächigen Elektroden als Messelektroden für den Blutstrom diese Differenzierung nicht möglich ist.

Ein wesentlicher Gedanke der Erfindung besteht darin, spezifisch den durch die Strömung des Blutes, das elektrochemisch als Elektrolyt zu betrachten ist, in einem Blutgefäß oder dem Herzen hervorgerufenen Effekt der konvektiven Diffusion auf ein in dort anliegendes elektrisches Signal zur Gewinnung einer Aussage über die Strömungsgeschwindigkeit auszunutzen. Weiter schließt die Erfindung den Gedanken ein, zu diesem Zwecke in das Blutgefäß Mikroelektroden zum Einbringen des besagten elektrischen (Anregungs-)Signals in die Strömungsbahn einzuführen und an eine geeignete Energiequelle anzuschließen. Schließlich ist an die genannten (oder auch andere) Mikroelektroden eine Signalerfassungseinrichtung zur Erfassung des besagten Effektes anzuschließen, und das erfasste elektrische Signal ist mit einem geeigneten Auswertungsverfahren auszuwerten.

Gegenüber bekannten Lösungen bietet die Erfindung wesentliche praktische Vorteile: So kann auf komplexe konstruktive Maßnahmen sowie bewegte Teile, wie sie in vielen Strömungsgeschwindigkeits-Fühlern vorhanden sind, vollständig verzichtet werden. Damit wird bereits im Ansatz verhindert, dass bei längerem Betrieb der Messeinrichtung Probleme durch Anlagerung von Bestandteilen des Blutes an bewegte Teile eines Messfühlers auftreten können. Des Weiteren kann gegenüber bekannten Verfahren mit kleiner Anregungsenergie gearbeitet werden, was einen vergleichsweise langen Betrieb der Vorrichtung als im Wesentlichen implantierbares Messgerät mit eingebauter Batterie ermöglicht.

Des Weiteren ist die vorgeschlagene Messvorrichtung in vorteilhafter Weise mit Komponenten implantierbarer Geräte zur Unterstützung von Körperfunktionen, speziell Herz-/ Kreislauf-Unterstützungsgeräten und den zu diesen gehörenden Elektrodenkabeln, kombinierbar. Hierdurch lassen sich wesentliche Kostenvorteile und Vorteile hinsichtlich des Einsetzens in den Körper eines Patienten erzielen.

Die Elektrodenanordnung der Messvorrichtung zeichnet sich in bevorzugten Ausführungen durch eine Miniaturisierung der Elektroden aus, um Messfehler zu minimieren. Diese Miniaturisierung ist mit Mitteln der Mikrostrukturtechnik, also mit aus der Halbleitertechnologie bekannten Strukturierungsmethoden, möglich und erlaubt im Übrigen eine Integration der Elektrodenanordnung mit der Signalerfassungseinrichtung oder zumindest Mitteln zur Signal-Vorverarbeitung auf ein und demselben Substrat (Chip).

Spezielle Ausführungen der Messvorrichtung enthalten mehrere Mikroelektrodenanordnungen, die voneinander unabhängig mit der Signalerfassungseinrichtung und optional auch mit der elektrischen Energiequelle verbunden sind und daher weitgehend selbstständige Sensoren darstellen können. Eine nach Möglichkeit weit gespreizte räumliche Verteilung solcher Anordnungen macht die Messvorrichtung insgesamt unempfindlicher gegen Einwachsvorgänge, indem eine aufgrund des Einwachsens nachlassende Sensorfunktion einer Mikroelektrodenanordnung durch die nach wie vor ungestörte Funktionalität anderer Mikroelektrodenanordnungen teilweise kompensiert wird. In einer Ausgestaltung dieser Ausführung ist eine Auswahlschaltung vorgesehen, die aufgrund eines externen Auswahlsignals oder der Signale einer internen Sensorik, die insbesondere direkt oder indirekt den Einwachszustand der einzelnen Elektrodenanordnungen erfassen kann, eine Auswahl der (noch) geeigneten Sensoren aktiviert. Das heißt, dass nicht taugliche Sensoren "abgeschaltet" werden.

In einer Ausführung der Erfindung ist vorgesehen, dass die Fläche der Mikroelektrodenanordnung im Bereich zwischen 100 und 10.000 µm², insbesondere zwischen 200 und 5.000 µm² und noch spezieller zwischen 300 und 2.000 µm² liegt. Wenn - was zur Gewinnung eines hinreichend hohen Summenstromes als Messsignal sinnvoll ist - eine größere Anzahl einzelner, kleinerer Mikroelektroden zu einem Array verschaltet wird, ist insbesondere vorgesehen, dass der laterale Abstand zwischen einzelnen Messelektroden der Mikroelektrodenanordnung mindestens das Dreifache, insbesondere mindestens das Fünffache und noch spezieller mindestens das Siebenfache der entsprechenden lateralen Abmessung der einzelnen Elektrode beträgt.

In einer ersten Ausführungsform sind die Mikroelektroden unipolar geschaltet, wobei etwa die Elektroden einer oder mehrerer parallel geschalteter Flächen einander (etwa auf dem Gerätegehäuse eines Herzschrittmachers oder auf dem indifferenten Pol einer Schrittmachersonde) gegenüber stehen. Eine hierzu alternative Ausführung sieht eine bipolare Verschaltung der einzelnen Mikroelektroden vor. Hierbei stehen sich etwa zwei einzelne Mikroelektrodenanordnungen oder eine solche Anordnung und eine gemeinsame Gegenelektrode gegenüber. In einer anderen Ausgestaltung der bipolaren Ausführung sind die Gegenpole jeweils in unmittelbarer Nähe der einzelnen Elektrodenpole angeordnet, und in einer im Interesse hoher Messgenauigkeit bevorzugten Ausführung ist zwischen ihnen ein möglichst langer und schmaler Spalt vorgesehen. Dies kann z.B. durch eine "koaxial" spiralige Anordnung von Elektrode und Gegenelektrode mit schmalem Spalt dazwischen, durch eine kammartige Verzahnung oder auch durch eine konzentrische Anordnung mit Ringspalt zwischen einer (bevorzugt sehr kleinen) inneren Elektrode und einer diese konzentrisch umgebenden äußeren Elektrode realisiert werden.

Ebenfalls im Sinne einer hohen Aussagekraft und Genauigkeit der Messwerte kann es vorteilhaft sein, wenn die Anregungs- und/oder Messelektroden eine mikrostrukturierte Oberfläche aufweisen, derart, dass die Kontaktfläche mit dem Blutstrom um ein Vielfaches größer ist als die geometrische Elektrodenfläche. Um die Messsignale möglichst weitgehend unabhängig von Störeinflüssen durch Herzaktionspotentiale zu machen, wird im Übrigen vorgeschlagen, dass die Messelektroden koaxial auf dem Leiterkabel angeordnet sind.

In einer zweckmäßigen Ausführung der Erfindung ist vorgesehen, dass die elektrische Energiequelle eine Wechselspannungsquelle und die Signalerfassungseinrichtung eine Messeinrichtung zur zeitabhängigen Messung einer komplexen Impedanz zwischen den Messelektroden aufweist. Alternativ hierzu kann die Messvorrichtung so arbeiten, dass die elektrische Energiequelle einen Impulsgenerator zur Erzeugung einer Anregungs-Impulsfolge und die Signalerfassungseinrichtung eine Messeinrichtung zur Erfassung einer an den Messelektroden auftretenden Impulsantwort aufweist.

In einer konstruktiven Ausführung der Erfindung ist vorgesehen, dass die Auswertungselektronik und optional auch die Anregungselektronik sich in unmittelbarer Nähe der Mikroelektrodenanordnung befindet und vorzugsweise dieser in einem Substrat integriert ausgeführt ist, so dass jegliche Leitungsverbindungen entfallen. So kann die Anregungs-energie etwa über eine Induktionsspule in die eigentliche Messvorrichtung eingekoppelt werden, die mit dieser baulich integriert ist. Für den Fall, dass in unmittelbarer räumlicher Nähe der Mikroelektrodenanordnung keine Signalerfassungseinrichtung platziert werden kann und dennoch auf Signalkabel verzichtet werden soll, kommt der Einsatz von als solchen bekannten Mitteln zur drahtlosen Signalübertragung, etwa eines so genannten RFID, in Betracht.

Merkmal einer weiteren Ausführung der Erfindung ist das Vorsehen von Leitungsverbindungen zwischen der Elektrodenanordnung und der Anregungs-Energiequelle einerseits sowie - in bevorzugten Ausführungen - der Signalerfassungseinrichtung und/oder der Auswertungseinrichtung andererseits. Hierbei ist nach obigem eine Ausführung möglich, bei der die Signalerfassungseinrichtung direkt mit der Elektrodenanordnung baulich verbunden und daher zwischen diesen keine an die Verlegung in einem Blutgefäß angepasste Leitung erforderlich ist; es kann dann aber eine Leitungsverbindung zwischen der Signalerfassungs- und -auswertungseinrichtung vorhanden sein, die im Blutgefäß verläuft.

Sinnvoll ist aus derzeitiger Sicht auch eine Ausführung, in der die elektrische Energiequelle und die Signalerfassungseinrichtung im Gehäuse eines implantierbaren Herz-/Kreislauf-Unterstützungsgerätes und die erste und optional zweite Leitungsverbindung in einem mit dem Unterstützungsgerät verbindbaren Leiterkabel und die Mikroelektrodenanordnung auf der Oberfläche des Leiterkabels angeordnet sind. Hierfür können in vorteilhafter Weise bekannte und bewährte Komponenten aus der Schrittmachertechnik Verwendung finden, welche um die Elektrodenanordnung und die elektronischen Komponenten zur Signalerfassung und -auswertung zu ergänzen sind.

Insbesondere kann dann vorgesehen sein, dass die elektrische Energiequelle und die Signalerfassungseinrichtung im Gehäuse eines Herzstimulationsgerätes und die Leitungsverbindung(en) und die Mikroelektrodenanordnung in bzw. auf einer Stimulationselektrodenleitung angeordnet sind. Auch die Signalauswertungseinrichtung kann bei dieser Ausführung im Gehäuse des Unterstützungsgerätes, speziell Herzschrittmachers oder Defibrillators oder eines Kombinationsgerätes, angeordnet sein.

Es ist jedoch auch möglich, die Signalauswertungseinrichtung außerhalb des Körpers des Patienten zu platzieren, um beispielsweise externe Überwachungen oder temporäre Untersuchungen seines hämodynamischen Zustandes zu realisieren. Sie kann dann etwa über eine Telemetriestrecke mit dem im Körper angeordneten Signalerfassungsteil verbunden sein, und über diese Telemetriestrecke - in bidirektionaler Ausführung - kann auch der Betrieb der Messvorrichtung gestartet und beendet und bedarfsweise hinsichtlich bestimmter Parameter gesteuert werden, wenn extrakorporal eine entsprechende Steuereinrichtung vorgesehen ist. Es versteht sich, dass bei der vollständig implantierten Ausführung eine derartige Steuereinrichtung im Gehäuse des implantierten Körperfunktions-Unterstützungsgerätes vorgesehen sein kann.

Umgekehrt wird bei einer derartigen integrierten Ausführung der Messvorrichtung mit einem Körperfunktions-Unterstützungsgerät eine Unterstützungs-Steuereinheit jenes Gerätes zur Steuerung seiner Assistenzfunktion eingangsseitig mit einem Ausgang der Signalauswertungseinrichtung der Messvorrichtung verbunden sein. So kann beispielsweise, falls ein Absinken der Strömungsgeschwindigkeit unter einen vorbestimmten Schwellwert festgestellt wird, auf eine lebensbedrohliche Anomalie des Herzrhythmus geschlossen und automatisch eine entsprechende antitachykarde Schrittmacherfunktion oder eine Defibrillatorfunktion des Unterstützungsgerätes ausgelöst werden.

Zur Gewinnung hinreichend verlässlicher Messwerte ist es zweckmäßig erforderlich, einerseits Maßnahmen zur Einhaltung eines Mindestabstandes zur Gefäßwandung zu treffen und andererseits (ungeachtet der relativen Unempfindlichkeit der vorgeschlagenen Vorrichtung in dieser Hinsicht) Vorsorge gegen Messwertverfälschungen durch auf die Elektroden aufwachsende Blutbestandteile zu treffen.

Der wünschenswerte Mindestbestand hängt bis zu einem gewissen Grade von der Fläche der eingesetzten Mikroelektroden ab und sollte nach bisherigem Erkenntnisstand im Bereich des Fünf- bis Zehnfachen der lateralen Erstreckung der Mikroelektroden liegen.

Ersteres kann durch das Vorsehen geeigneter Abstandshalterelemente an der Elektrodenanordnung realisiert werden, die zur Erleichterung eines Einführens in das Blutgefäß zweckmäßigerweise ein- und ausfahrbar bzw. (bevorzugt elastisch) abspreiz- und anklappbar ausgeführt sind. Letzteres wird durch Wahl einer geeigneten metallischen Oberfläche, die das Aufwachsen von biologischem Material nicht begünstigt, oder ggf. durch Vorsehen einer spezifisch aufwachshemmenden Beschichtung realisiert.

Wesentliche Aspekte eines erfindungsgemäßen Messverfahrens ergeben sich zwanglos aus den oben erwähnten Aspekten der erfindungsgemäßen Vorrichtung bzw. bevorzugter Ausführungen derselben, so dass diese Verfahrensaspekte hier nicht wiederholt werden. Erwähnt werden soll jedoch, dass in zwei grundsätzlichen Alternativen die Bereitstellung der Anregungsenergie voltammetrisch oder potentiometrisch erfolgt, die Messung also als Erfassung von Strom- oder Spannungssignalen erfolgt.

Des Weiteren wird darauf hingewiesen, dass die Anregungsenergie für den Messvorgang in Abstimmung auf die relevanten Reizbildungsprozesse im Körper des Patienten zu erfolgen hat, um Störungen seiner Körperfunktionen sicher auszuschließen. Insbesondere sind hierfür obere Spannungs- bzw. Strom-Grenzwerte zu beachten, die eine Beeinflussung der Herzaktionspotentiale sicher ausschließen. Hierzu wird es in der Regel ausreichend sein, dass die Amplitude einer Anregungsspannung oder eines Anregungsstromes maximal die Hälfte einer Stimulationsschwelle des menschlichen Herzens beträgt, insbesondere unter 50 mV liegt bzw. mit dem Auftreten einer Spannung von weniger als 50 mV verbunden ist.

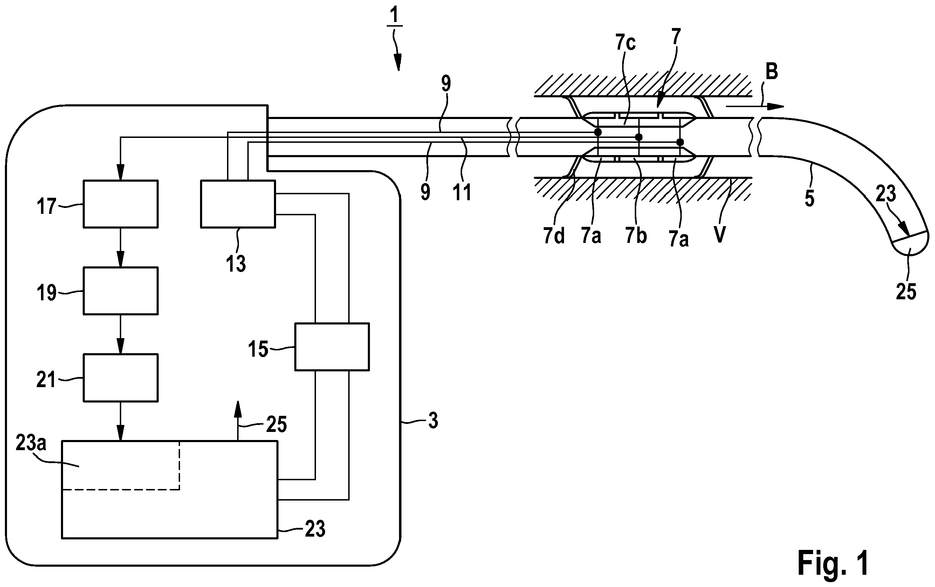

Vorteile und Zweckmäßigkeiten der Erfindung ergeben sich im Übrigen aus der Beschreibung eines Ausführungsbeispiels anhand der einzigen Figur. Diese zeigt - in skizzenartiger nicht maßstäblicher Darstellung in Art eines Funktions-Blockschaltbildes - eine vollständig implantierbare Messvorrichtung 1, welche baulich weitgehend mit einem Antitachykardie-Herzschrittmacher 3 bzw. einer mit diesem verbundenen Stimulationselektrodenleitung 5 integriert ist, welche abschnittsweise in einem Blutgefäß V verläuft, in dem die Strömungsgeschwindigkeit eines Blutstromes B gemessen werden soll. Der Herzschrittmacher 3 und die Elektrodenleitung 5 sind in der Figur nur insoweit dargestellt, als die Beschreibung der Ausführungsform der Erfindung dies erfordert.

Die Messvorrichtung 1 umfasst eine Mikroelektrodenanordnung 7, die koaxial auf dem Umfang der Elektrodenleitung 5 angeordnet ist und jeweils mehrere Anregungselektroden 7a und Messelektroden 7b, in Mikrostrukturtechnik realisiert auf einem Substrat 7c, umfasst. Der Elektrodenanordnung 7 sind mehrere um den Umfang der Elektrodenleitung 5 verteilte Abstandshalter 7d zugeordnet, die einen zur Vermeidung von MesswertVerfälschungen hinreichenden Abstand der Elektroden von der Wandung des Blutgefäßes V sicherstellen. Um die Elektrodenleitung trotz dieser Abstandshalter leicht verlegen zu können, sind sie unter einem spitzen Winkel zur Längsachse der Elektrodenleitung und elastisch an diese andrückbar ausgeführt.

An die Anregungselektroden 7a wird über Versorgungsleitungen 9 eine elektrische Anregungsenergie (Wechsel- oder Impulsspannung) angelegt, und von den Messelektroden 7b wird über Messleitungen (hier dargestellt als eine einzelne Leitung 11) ein durch die Anregungs-Energie induziertes (Roh-)Messsignal abgegriffen. Die Anregungsenergie wird in einer Anregungs-Energiequelle 13 erzeugt, die von einer Schrittmacherbatterie 15 gespeist wird. Die Anregungs-Energiequelle 13 erzeugt aus der durch die Batterie 15 bereitgestellten Betriebsspannung ein Wechselspannungs- oder Impulssignal geringer Amplitude, etwa zwischen 10 mV und 50 mV, welches jedoch zur Induzierung eines Messsignals ausreicht.

Das Messsignal wird einer Signalerfassungsstufe 17 zugeführt, in der ein Summensignal aus den an den einzelnen Messelektroden anfallenden Messwerten gebildet und im Falle der Anregung durch eine Wechselspannung das auftretende Wechselspannungs-Signal oder im Falle der Anregung durch eine Impulsspannung die induzierte Impulsantwort als zeitabhängige Größe erfasst wird. Ausgangsseitig ist die Signalerfassungsstufe 17 mit einer Auswertungsstufe 19 verbunden, in der anhand eines vorgespeicherten Auswertungsalgorithmus aus den erfassten elektrischen Messsignalen zugehörige Blutströmungs-Geschwindigkeitswerte ermittelt werden.

Ausgangsseitig ist die Auswertungsstufe 19 ihrerseits mit einer Schwellwertdiskriminatorstufe 21 verbunden, in der aufgrund vorgespeicherter Blutgeschwindigkeits-Schwellwerte eine Detektierung etwaiger unzulässig niedriger Geschwindigkeitswerte erfolgt, die auf eine hämodynamisch bedenkliche Situation beim Patienten schließen lassen. Die Schwellwertdiskriminatorstufe 21 ihrerseits ist ausgangsseitig mit einem Steuereingang einer Stimulations-Steuerstufe 23a einer Schrittmacherelektronik 23 verbunden. Erzeugt die Diskriminatorstufe 21 ein Signal, welches einen Blutstillstand oder eine unzulässig niedrige Blutgeschwindigkeit anzeigt, leitet die Stimulations-Steuerstufe 23a die Ausgabe einer Stimulations-Impulsfolge an eine Stimulationselektrode 25 an der Spitze der Elektrodenleitung 5 ein. (Die Signalverbindung zwischen beiden Komponenten der Schrittmacheranordnung 3/5 ist in der Figur aus Gründen der besseren Übersichtlichkeit nicht durchgehend gezeichnet.)

Die Ausführung der Erfindung ist nicht auf dieses Beispiel und die oben hervorgehobenen Aspekte beschränkt, sondern ebenso in einer Vielzahl von Abwandlungen möglich, die im Rahmen fachgemäßen Handelns liegen.

## Patentansprüche

1. Vorrichtung (1) zur in-vivo-Erfassung der Strömungsgeschwindigkeit eines Blutstromes (B) im Blutkreislauf (V), mit
- einer Mikroelektrodenanordnung, die zur Platzierung im Blutkreislauf eines Patienten ausgebildet ist und welche bipolar geschaltete Mikroelektroden aufweist,
- einer elektrischen Energiequelle (13), die zur Bereitstellung von Anregungs-energie zur Gewinnung eines Messsignals ausgebildet und mit der Mikroelektrodenanordnung verbunden ist,
- einer Signalerfassungseinrichtung (17) zur Erfassung eines durch den Blutstrom in Gegenwart der Anregungsenergie an Messelektroden (7b) der Mikroelektrodenanordnung entstehenden elektrischen Messsignals und
- einer eingangsseitig mit der Signalerfassungseinrichtung verbundenen Signalauswertungseinrichtung (19) zur Bestimmung der Strömungsgeschwindigkeit aus dem Messsignal,
**dadurch gekennzeichnet, dass**
die Elektrode als Elektrodenpol und deren Gegenelektrode als Gegenpol durch eine konzentrische Anordnung mit Ringspalt zwischen einer inneren Elektrode und einer diese konzentrisch umgebenden äußeren Elektrode ausgebildet ist.

2. Vorrichtung nach Anspruch 1, **dadurch gekennzeichnet, dass** die elektrische Energiequelle (13)
- eine Wechselspannungsquelle und die Signalerfassungseinrichtung eine Messeinrichtung zur zeitabhängigen Messung einer komplexen Impedanz zwischen den Messelektroden aufweist oder
- einen Impulsgenerator zur Erzeugung einer Anregungs-Impulsfolge und die Signalerfassungseinrichtung eine Messeinrichtung zur Erfassung einer an den Messelektroden auftretenden Impulsantwort aufweist.

3. Vorrichtung nach einem der vorangehenden Ansprüche, **dadurch gekennzeichnet, dass** die Elektrodenanordnung (7) Mikroelektroden, bevorzugt in integrierter Schaltungsanordnung mit der Signalerfassungseinrichtung, aufweist.

4. Vorrichtung nach einem der vorangehenden Ansprüche, **dadurch gekennzeichnet, dass** mindestens ein Teil der Anregungselektroden zugleich Messelektroden sind.

5. Vorrichtung nach einem der vorhergehenden Ansprüche, **dadurch gekennzeichnet, dass** die Anregungs- und/oder Messelektroden (7a, 7b) eine mikrostrukturierte Oberfläche aufweisen, derart, dass die Kontaktfläche mit dem Blutstrom um ein Vielfaches größer ist als die geometrische Elektrodenfläche.

6. Vorrichtung nach einem der vorangehenden Ansprüche, **dadurch gekennzeichnet, dass** die Fläche der Mikroelektrodenanordnung im Bereich zwischen 100 und 10.000 µm², bevorzugt zwischen 200 und 5.000 µm² und besonders bevorzugt zwischen 300 und 2.000 µm² liegt.

7. Vorrichtung nach einem der vorhergehenden Ansprüche, **dadurch gekennzeichnet, dass** der laterale Abstand zwischen einzelnen Messelektroden (7b) der Mikroelektrodenanordnung mindestens das Dreifache, bevorzugt mindestens das Fünffache und besonders bevorzugt mindestens das Siebenfache der entsprechenden lateralen Abmessung der einzelnen Elektrode beträgt.

8. Vorrichtung nach einem der vorangehenden Ansprüche, mit mindestens einer zur Verlegung in dem Blutgefäß ausgebildeten Leitungsverbindung (11) zwischen der Energiequelle (13) und Anregungselektroden (7a) der Mirkoelektrodenanordnung und zwischen Messelektroden der Mikroelektrodenanordnung (7) und der Signalerfassungseinrichtung (17) oder der Signalerfassungseinrichtung und der Signalauswertungseinrichtung.

9. Vorrichtung nach einem Anspruch 8, **dadurch gekennzeichnet, dass** die elektrische Energiequelle (13) und die Signalerfassungseinrichtung (17) im Gehäuse eines implantierbaren Herz-/Kreislauf-Unterstützungsgerätes (3) und die oder jede Leitungsverbindung (9, 11) in einem mit dem Unterstützungsgerät verbindbaren Leiterkabel (5) und die Elektrodenanordnung (7) auf der Oberfläche des Leiterkabels angeordnet sind und wobei die elektrische Energiequelle (13) und die Signalerfassungseinrichtung (17) im Gehäuse eines Herzstimulationsgerätes (3) und die oder jede Leitungsverbindung (9, 11) und die Mikroelektrodenanordnung (7) in bzw. auf einer Stimulationselektrodenleitung (5) angeordnet sind.

10. Vorrichtung nach einem der vorangehenden Ansprüche, mit mehreren Mikroelektrodenanordnungen, die jeweils unabhängig voneinander mit der Signalerfassungseinrichtung und optional auch unabhängig voneinander mit der Energiequelle verbunden sind, derart, dass sie unabhängig gewonnene Messsignale liefern.

11. Vorrichtung nach Anspruch 8, **dadurch gekennzeichnet, dass** den mehreren Mikroelektrodenanordnungen eine Auswahlschaltung zugeordnet ist, die eine oder mehrere Anordnungen aus der Mehrzahl in Abhängigkeit von einem der Vorrichtung von extern zugeführten Auswahlsignal oder vom Signal eines der Vorrichtung intern zugeordneten Sensors, der insbesondere direkt oder indirekt einen Einwachszustand der einzelnen Mikroelektrodenanordnungen erkennt, aktiviert, bevorzugt ist die Mehrzahl der Mikroelektrodenanordnungen räumlich getrennt auf einem Substrat, positioniert.

12. Vorrichtung nach einem der vorangehenden Ansprüche, **dadurch gekennzeichnet, dass** die Energiequelle und/oder die Signalerfassungseinrichtung der Mikroelektrodenanordnung integriert mit der Mikroelektrodenanordnung ausgeführt ist oder in drahtloser Wirkverbindung mit ihr steht.

13. Vorrichtung nach einem der vorangehenden Ansprüche, **dadurch gekennzeichnet, dass** der Elektrodenanordnung Abstandshaltermittel (7d) zur Einhaltung eines vorbestimmten Mindestabstandes zur Wandung des Blutgefäßes im Betrieb der Vorrichtung zugeordnet sind, welche bevorzugt elastisch abspreiz- und andrückbar oder aus einer Oberfläche der Elektrodenanordnung ausfahrbar ausgebildet sind.

14. Herz-/Kreislauf-Unterstützungsgerät mit einer Vorrichtung nach einem der vorangehenden Ansprüche sowie einer Unterstützungs-Steuereinrichtung zur Steuerung der Assistenzfunktion, welche eingangsseitig mit einem Ausgang der Signalauswertungseinrichtung verbunden ist, wobei das Herz-/Kreislauf-Unterstützungsgerät vorzugsweise als implantierbarer elektrischer Herzschrittmacher und/oder Defibrillator ausgebildet ist.

## Claims

1. A device (1) for in vivo detection of the flow velocity of a blood stream (B) in the blood circulation (V), with
- a microelectrode arrangement that is designed for placement in the circulation of a patient and whose microelectrodes have a bipolar configuration,
- an electrical energy source (13), that is designed to provide stimulation energy to obtain a measurement signal and that is connected with the microelectrode arrangement,
- a signal detection device (17) to detect an electrical measurement signal on measuring electrodes (7b) of the microelectrode arrangement, this electrical measurement signal being produced by the blood stream in the presence of the stimulation energy; and
- a signal evaluation device (19) whose input side is connected with the signal detection device to determine the flow velocity from the measurement signal,
**characterized in that**
the electrode in the form of an electrode pole and its counter electrode in the form of a counter pole are formed by a concentric arrangement with an annular gap between an inner electrode and an outer electrode concentrically surrounding this inner electrode.

2. A device according to claim 1, **characterized in that** the electrical energy source (13)
- has an alternating voltage source and the signal detection device has a measuring device for time-dependent measurement of a complex impedance between the measuring electrodes; or
- has a pulse generator to produce a stimulation pulse train and the signal detection device has a measuring device to detect a pulse response on the measuring electrodes.

3. A device according to any one of the preceding claims, **characterized in that** the electrode arrangement (7) has microelectrodes, preferably in an integrated circuit arrangement with the signal detection device.

4. A device according to any one of the preceding claims, **characterized in that** at least part of the stimulation electrodes are simultaneously measuring electrodes.

5. A device according to any one of the preceding claims, **characterized in that** the stimulation and/or measuring electrodes (7a, 7b) have a microstructured surface, so that the contact surface with the blood stream is multiple times larger than the geometric surface of the electrode.

6. A device according to any one of the preceding claims, **characterized in that** the surface area of the microelectrode arrangement lies in the range between 100 and 10,000 µm², preferably between 200 and 5,000 µm², and especially preferably between 300 and 2,000 µm².

7. A device according to any one of the preceding claims, **characterized in that** the lateral distance between individual measuring electrodes (7b) of the microelectrode arrangement is at least three times, preferably at least five times, and especially preferably at least seven times the corresponding lateral dimension of the individual electrode.

8. A device according to any one of the preceding claims, with at least one lead connection (11) that is designed to be deployed in the blood vessel, this lead connection (11) running between the energy source (13) and stimulation electrodes (7a) of the microelectrode arrangement and between measuring electrodes of the microelectrode arrangement (7) and the signal detection device (17) or the signal detection device and the signal evaluation device.

9. A device according to claim 8, **characterized in that** the electrical energy source (13) and the signal detection device (17) are arranged in the housing of an implantable cardiovascular support device (3) and the or every lead connection (9, 11) is/are arranged in a conductor cable (5) that is connectable with the support device and the electrode arrangement (7) is arranged on the surface of the conductor cable, the electrical energy source (13) and the signal detection device (17) being arranged in the housing of a cardiac stimulation device (3) and the or every lead connection (9, 11) and the microelectrode arrangement (7) being arranged in or on a stimulation electrode lead (5).

10. A device according to any one of the preceding claims, with multiple microelectrode arrangements, each of which is connected with the signal detection device independently of the others and optionally is also connected with the energy source independently of the others, so that they supply measurement signals that they obtain independently of one another.

11. A device according to claim 8, **characterized in that** the multiple microelectrode arrangements are associated with a selection circuit, which activates one or more of the multiple microelectrode arrangements depending on a selection signal supplied to the device from outside or on the signal of a sensor internally associated with the device, this sensor in particular directly or indirectly recognizing an ingrowth state of the individual microelectrode arrangements, the multiple microelectrode arrangements preferably being positioned on a substrate so that they are spatially separated.

12. A device according to any one of the preceding claims, **characterized in that** the energy source and/or the signal detection device of the microelectrode arrangement is/are designed to be integrated with the microelectrode arrangement or has/have a wireless connection with it.

13. A device according to any one of the preceding claims, **characterized in that** the electrode arrangement is associated with spacing means (7d) to maintain a predetermined minimum spacing to the wall of the blood vessel when the device is in operation, these spacing means (7d) preferably being designed so that they can be elastically braced and pressed or extended from a surface of the electrode arrangement.

14. A cardiovascular support device with a device according to any one of the preceding claims and a support controller for controlling the assistance function, the input side of this support controller being connected with an output of the signal evaluation device, the cardiovascular support device preferably being in the form of an implantable electrical cardiac pacemaker and/or defibrillator.

## Revendications

1. Dispositif (1) destiné à la détection in vivo de la vitesse d'écoulement d'un flux sanguin (B) dans la circulation sanguine (V) doté
- d'un ensemble de microélectrodes qui est conçu pour la mise en place dans la circulation sanguine d'un patient et lequel présente des microélectrodes branchées de manière bipolaire,
- d'une source d'énergie électrique (13) qui est conçue pour la fourniture d'une énergie d'excitation pour l'élaboration d'un signal de mesure et est reliée avec l'ensemble de microélectrodes,
- d'un dispositif de détection de signal (17) pour la détection d'un signal de mesure électrique engendré par le flux sanguin en présence de l'énergie d'activation à des électrodes de mesure (7b) de l'ensemble de microélectrodes, et
- d'un dispositif de traitement de signal (19) relié du côté entrée avec le dispositif de détection de signal pour la détermination de la vitesse d'écoulement à partir du signal de mesure,
**caractérisé en ce que**
l'électrode est conçue en tant que pôle d'électrode et sa contre-électrode est conçue en tant que pôle complémentaire par un ensemble concentrique avec une fente annulaire entre une électrode interne et une électrode externe entourant celle-ci de manière concentrique.

2. Dispositif selon la revendication 1, **caractérisé en ce que** la source d'énergie électrique (13)
- présente une source de tension variable et le dispositif de détection de signal présente un dispositif de mesure pour la mesure en fonction du temps d'une impédance complexe entre les électrodes de mesure, ou
- un générateur d'impulsions pour la génération d'une suite d'impulsions d'excitation et le dispositif de détection de signaux présente un dispositif de mesure pour la détection d'une réponse d'impulsion se produisant au niveau des électrodes de mesure.

3. Dispositif selon l'une des revendications précédentes, **caractérisé en ce que** l'ensemble d'électrodes (7) présente des microélectrodes, de préférence, dans un ensemble de circuit avec un dispositif de détection de signal.

4. Dispositif selon l'une des revendications précédentes, **caractérisé en ce qu'**au moins une partie des électrodes d'excitation constitue simultanément un ensemble d'électrodes de mesure.

5. Dispositif selon l'une des revendications précédentes, **caractérisé en ce que** les électrodes d'excitation et/ou de mesure (7a, 7b) présentent une surface micro-structurée de telle manière que la surface de contact avec le flux sanguin est supérieure d'un multiple de fois à la surface d'électrode géométrique.

6. Dispositif selon l'une des revendications précédentes, **caractérisé en ce que** la surface de l'ensemble de microélectrodes se situe dans la plage entre 100 et 10 000 µm², de préférence entre 200 et 5 000 µm² et de manière particulièrement préférée entre 300 et 2 000 µm².

7. Dispositif selon l'une des revendications précédentes, **caractérisé en ce que** la distance latérale entre des électrodes de mesure (7b) individuelles de l'ensemble de microélectrodes s'élève à au moins trois fois, de préférence, à au moins cinq fois, et de manière particulièrement préférée à au moins sept fois, la dimension latérale correspondante de l'électrode individuelle.

8. Dispositif selon l'une des revendications précédentes, avec au moins une connexion de ligne (11) conçue pour un déplacement dans le vaisseau sanguin entre la source d'énergie (13) et les électrodes d'excitation (7a) de l'ensemble de microélectrodes et entre des électrodes de mesure de l'ensemble de microélectrodes (7) et le dispositif de détection de signal (17), ou du dispositif de détection de signal et du dispositif de traitement de signal.

9. Dispositif selon la revendication 8, **caractérisé en ce que** la source d'énergie électrique (13) et le dispositif de détection de signal (17) sont disposés dans le boîtier d'un appareil d'assistance pour le coeur et la circulation (3) implantable, et la ou une certaine connexion de ligne (9, 11) est disposée dans un câble conducteur (5) pouvant être relié avec l'appareil d'assistance et l'ensemble d'électrodes (7) sur la surface du câble conducteur, et où la source d'énergie électrique (13) et le dispositif de détection de signal (17) sont disposés dans le boîtier d'un appareil de stimulation cardiaque (3) et la ou une certaine connexion de ligne (9, 11) et l'ensemble de microélectrodes (7) sont disposés respectivement sur une ligne d'électrode de stimulation (5).

10. Dispositif selon l'une des revendications précédentes, avec plusieurs ensembles de microélectrodes qui sont reliés respectivement de manière indépendante les uns par rapport aux autres avec le dispositif de détection de signal et éventuellement également de manière indépendante les uns par rapport aux autres avec la source d'énergie, de telle manière qu'ils délivrent des signaux de mesure reçus de manière indépendante.

11. Dispositif selon la revendication 8, **caractérisé en ce qu'**une commutation de sélection est associée aux nombreux ensembles de microélectrodes, qui active un ou plusieurs ensembles de la pluralité en fonction d'un signal de sélection apporté de l'extérieur du dispositif ou du signal d'un capteur associé de manière interne au dispositif, qui reconnaît en particulier directement ou indirectement un état d'implantation des ensembles de microélectrodes individuels, de préférence la pluralité des ensembles de microélectrodes est séparée dans l'espace positionnée sur un substrat.

12. Dispositif selon l'une des revendications précédentes, **caractérisé en ce que** la source d'énergie et/ou le dispositif de détection de signal de l'ensemble de microélectrodes est conçu(e) intégré(e) avec l'ensemble de microélectrodes ou est en liaison avec celui-ci par une liaison active sans fil.

13. Dispositif selon l'une des revendications précédentes, **caractérisé en ce que** des moyens d'espacement (7d) sont associés à l'ensemble d'électrodes pour le maintien d'une distance minimale prédéterminée par rapport à la paroi du vaisseau sanguin pendant le fonctionnement du dispositif, lesquels sont conçus de préférence extensibles élastiquement et compressibles ou peuvent conçus pour sortir d'une surface de l'ensemble d'électrodes.

14. Appareil d'assistance pour le coeur et la circulation doté d'un dispositif selon l'une des revendications précédentes, ainsi que d'un dispositif de commande de l'assistance pour la commande de la fonction d'assistance, lequel est relié du côté entrée avec une sortie du dispositif de traitement de signal, où l'appareil d'assistance pour le coeur et la circulation est conçu de préférence en tant que stimulateur cardiaque et/ou que défibrillateur électrique implantable.
